# EUROPÄISCHE PATENTANMELDUNG

(11) **EP 1 623 685 A1**
(43) Veröffentlichungstag der Anmeldung: **08.02.2006**
(21) Anmeldenummer: 04018714.8
(22) Anmeldetag: 06.08.2004
(51) Int. Cl.: A61F 2/36

(54) **Hüftgelenkprothese mit einem in den Oberschenkelknochen einzusetzenden Schaft**

(71) Anmelder: WALDEMAR LINK GmbH & Co. KG, 22339 Hamburg (DE)
(72) Erfinder: Link, Helmut D., 22397 Hamburg (DE)
(74) Vertreter: Glawe, Delfs, Moll

(57) **Zusammenfassung**

Hüftgelenkprothese mit einem in den Oberschenkelknochen einzusetzenden Schaft (3), dessen Oberfläche eine osteoinduktive Ausrüstung aufweist. Diese Ausrüstung ist ausschließlich im metaphysären Abschnitt (5) des Schafts (3) und lateral von der Linie (7) größter AP-Abmessung des Schaftquerschnitts vorgesehen. Erreicht wird dadurch eine bessere Einbeziehung der metaphysären Spongiosa in den Kraftfluß, ohne daß die Reoperierbarkeit der Prothese beeinträchtigt wird.

## Beschreibung

Das spongiöse Knochengewebe in der Metaphyse des Oberschenkelknochens weist eine komplizierte Struktur aus Knochenbälkchen auf, die die druck- und zugbelasteten Teile des Knochens am Schenkelhals, dem großen Trochanter, dem kleinen Trochanter und der Diaphyse druck- und zugübertragend verbinden. In ihrer Summe bilden sie durchgehende Zug- und Druck-Trajektorien (Farbatlanten der Medizin, Bd 7: Bewegungsapparat I. Verlag Thieme, Stuttgart, 1992). Wenn der Schaft einer Hüftgelenkprothese eingesetzt wird, werden insbesondere die primären Zugtrajektorien, die den Schenkelhals mit dem gegenüberliegenden, intertrochantären Oberflächenbereich des Knochens verbinden, großenteils unterbrochen. Wenn sie anschließend nicht mehr an der Kraftübertragung beteiligt sind, bilden sie sich zurück. Dies gilt insbesondere bei Verwendung solcher Prothesen, deren Prothesenschaft in der Diaphyse verklemmt wird und bei denen der proximale, metaphysäre Bereich des Oberschenkelknochens besonders in seinem lateralen Teil kaum in die Kraftübertragung einbezogen wird. Man hat versucht, durch sogenannte Zuganker den Prothesenschaft mit dem Bereich des großen Trochanters zu verbinden und diesen dadurch in den Kräftefluß einzubeziehen. Dabei wurde eine mit dem Prothesenschaft verbundene Stange durch den großen Trochanter geführt und außen mit einer Kontermutter versehen, so daß bei Belastung der Hüftprothese ein Zug auf den großen Trochanter ausgeübt wird (US-A-3,995,323, EP-B-93230, DE-B-1943598). Es hat sich aber gezeigt, daß derartige mechanische Zuganker in Folge der ständigen Wechselbelastung schnell locker werden und deshalb nur kurzfristig wirken. Ferner ist es bekannt, den Schaft oder einen davon lateral in den Bereich des großen Trochanters vorragenden Flügel so auszubilden, daß sich eine innige Verbindung mit der in Poren oder Öffnungen dieses Flügels einwachsenden Knochensubstanz ergibt (GB-A-1030145, FR-A-2356465, EP-A-128036, EP-A-222236, EP-A-95440, EP-B-601223, EP-A-1044665, US-A-5755811, US-A-4718915, US-A-5370698, FR-C-2194123). Um die Verbindung des Knochens mit der Prothesenoberfläche zu fördern, ist es auch bekannt, die Prothesenoberfläche osteokonduktiv zu gestalten. So bezeichnet man Oberflächen, die das benachbarte Knochenwachstum dulden. Dazu gehören Oberflächen aus Titanlegierungen sowie Beschichtungen, die Kalziumphosphat oder Hydroxylapatit enthalten (EP-A-761182, WO9308771).

In jüngerer Zeit sind Substanzen bekannt geworden, die das Knochenwachstum nicht nur wie die osteokonduktiven Oberflächen dulden, sondern undifferenzierte pluripotente Stammzellen zur Umbildung in Knochenzellen stimulieren (Albrechtsson, Johansson: Osteoinduction, Osteoconduction and Osseointegration; In: Gunzburg Hrsg.: The use ob bone substitutes in Spine Surgery; Springer. - Denissen, H. et al.: Ceramic hydroxyapatite Implants for the Release of Bisphosphonate; in: Bone and Mineral 1994, S. 123-134. - Yoshinari, M. et al.: Bone response sto calcium phosphatecoated and bisphosphonate-immobilized titanium implants; in: Biomaterials 2002 S. 2879-2885. - Yoshinari, M. et al.: Immobilization of bisphosphonates on surface modified titanium; in: Biomaterials 2001 S. 709-715). Zu diesen Substanzen gehören Bisphosphonate und Bone Morphogenic Proteins (kurz: BMP). Diese kann man auch zur Ausrüstung der Oberflächen von Knochenprothesen, einschließlich Hüftprothesen, verwenden (US-A-2002/0049497, US-A-2002/0127261). Sie führen zu einer sehr innigen Verbindung der Prothesenoberfläche mit dem Knochen, die im Falle einer Reoperation unerwünscht sein kann, weil dadurch die Lösung der zu entfernenden Prothese vom Knochen behindert werden kann.

Der Erfindung liegt die Aufgabe zugrunde, die Einbindung einer Oberschenkel-Hüftprothese in den Knochen zu verbessern, ohne die Reoperierbarkeit zu gefährden. Die erfindungsgemäße Lösung besteht in den Merkmalen der Ansprüche.

Demnach ist vorgesehen, daß bei einer Hüftgelenkprothese mit einem in den Oberschenkelknochen einzusetzenden Schaft die osteoinduktive Beschichtung ausschließlich in einem lateral von der Linie größter AP-Abmessung im metaphysären Abschnitt liegenden Teil des Schafts vorgesehen ist.

In einer älteren, nicht zum vorveröffentlichten Stand der Technik gehörenden Patentanmeldung (PCT/EP03/05292) wurde vorgeschlagen, die Ausrüstung mit der osteoinduktiven Substanz ausschließlich im trochantären Bereich vorzusehen, der folgendermaßen definiert ist. Geht man vom Schnittpunkt zwischen der Schenkelhals-Mittellinie mit der Mittellinie des proximalen Diaphysen-Endes aus, so liegt der trochantäre Bereich lateral von der von diesem Schnittpunkt gezogenen Tangente an den oberen Rand des Hüftkopfs und lateral von den diese Tangente fortsetzenden Teil der Schenkelhals-Mittellinie.

Gemäß der vorliegenden Erfindung wird die Grenze des mit der osteoinduktiven Substanz auszurüstenden Oberflächenbereichs anders definiert. Die erfindungsgemäße Definition läuft darauf hinaus, daß die osteoinduktive Ausrüstung der Prothesenoberfläche in demjenigen Teil des metaphysären Abschnitts vorgenommen wird, in welchem besonders viele Flächenanteile vorkommen, deren Richtungsnormale eine laterale Komponente enthält. In diesen Flächenanteilen kann nach bisheriger Erfahrung keine zugkraftübertragende Verbindung mit dem Knochen erwartet werden. Dank der osteoinduktiven Beschichtung wird eine solche Verbindung jedoch größtenteils ermöglicht.

Besonders zweckmäßig ist es wenn die Substanz in eine Beschichtung eingebracht wird, die außerdem porös sein sollte. Die Beschichtung kann von beliebiger Art sein. Beispielsweise kann es sich um einen poröse Metallschicht handeln. Besonders vorteilhaft sind solche Beschichtungen, die von Haus aus osteokonduktiv sind und beispielsweise aus Kalziumphosphat oder Hydroxylapatit bestehen.

Die Wirkung der Erfindung besteht darin, daß sehr rasch nach der Operation Knochenzellen in unmittelbarer Nachbarschaft und Verbindung zur Prothesenoberfläche entstehen. Damit wird erreicht, daß sich nicht infolge Relativbewegungen zwischen der Knochenoberfläche und dem Knochen zunächst ein Spalt oder eine bindegewebige Zwischenschicht bildet, die den späteren innigen Verbund beeinträchtigt oder unmöglich macht. Dank der Erfindung findet an der trochantären Oberfläche der Prothese eine raschere Knochenanlagerung und ein Knocheneinbau in deren Poren und Vertiefungen statt, so daß der trochantäre Knochenbereich rasch eine dauerhafte Verbindung mit der Prothese erhält und als Folge davon an der Kraftübertragung beteiligt wird. Hingegen findet an den übrigen Oberflächenteilen die Verbindung mit dem Knochen nur in dem auch bisher schon gewohntem Maße statt. Außerhalb des trochantären Bereichs, der im Falle einer Reoperation gut zugänglich ist und daher auch bei einer sehr starken Knochenbindung keine Probleme macht, findet der Arzt daher im Falle einer Reoperation genau diejenigen Verhältnisse vor, mit denen er bereits vertraut ist.

Der die osteoinduktive Substanz enthaltende Teil der Oberfläche weist zweckmäßigerweise Poren oder Hinterschnitte in bezug auf die Lateralrichtung auf, damit die sich aufgrund der Osteoinduktion gebildete Knochensubstanz nicht nur durch Haftung an der Oberfläche sondern auch durch Formschluß daran verankern kann.

In der Mitte des trochantären Teils des Knochens ist die Spongiosa mitunter weniger dicht ausgebildet, als nahe der Knochenrinde. Vorzugsweise befinden sich deshalb die nach ventral und dorsal gewendeten Anteile der osteoinduktiv ausgerüsteten Oberflächenanteile der Prothese in einem gewissen Abstand von der Mittelebene des Knochens und näher der Knochenrinde. Deshalb soll der diese Oberflächenanteile bildende Teil der Prothese in AP-Richtung nicht zu dünn sein. Seine Dicke und damit der Abstand der genannten dorsal bzw. ventral gelegenen Oberflächenanteile voneinander liegt zweckmäßigerweise über 6 und weiter vorteilhaft über 9 mm bis etwa 15 mm.

Das Anwachsen frischer Knochenzellen an der Prothesenoberfläche kann durch einen Preßsitz der betreffenden Flächenanteile gefördert werden. Zweckmäßig ist es deshalb, die betreffenden Flächen und deren Gegenflächen in der Richtung, in welcher die Prothese in den Knochen eingefügt wird, keilförmig auszuführen und die der Prothese zugehörige Raspel, die dazu benutzt wird, den Aufnahmeraum für den Prothesenschaft zu formen, mit knapperen Querschnittsmaßen auszustatten, so daß beim Einschieben des Prothesenschafts in den durch die Raspel vorbereiteten Raum die betreffenden Flächenanteile Knochenmaterial verdrängen.

Die Erfindung wird im folgenden näher unter Bezugnahme auf die Zeichnung erläutert. Darin zeigen:
- Fig. 1: eine Ventralansicht einer femuralen Hüftgelenkprothese,
- Fig. 2: einen Schnitt Längslinie II-II der Fig. 1,
- Fig. 3 und 4: alternative Querschnittsformen,
- Fig. 5: einen Schnitt Längslinie V-V der Fig. 1.

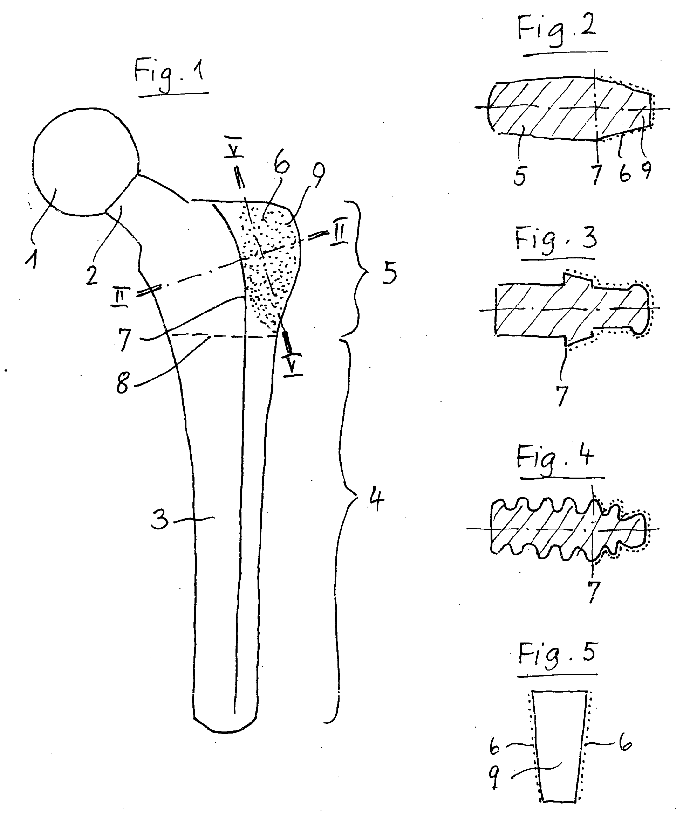

Die Hüftprothese gemäß Fig. 1 umfaßt einen Gelenkkopf 1, einen Hals 2, der einen Schaft 3 aufweist. Dieser besitzt einen diaphysären, in der Diaphyse des Knochens zu verankernden Abschnitt 4 und einen metaphysären, in der Metaphyse des Knochens zu verankernden Abschnitt 5. Der diaphysäre Abschnitt ist so bemessen, daß er eine primäre Verankerung des Schafts in der Diaphyse des Oberschenkelknochens bewirkt. Der Fachmann erkennt bei Betrachtung der Fig. 1, wie die Prothese im Knochen liegen wird, und weiß daher auch, wo die Grenze 8 zwischen dem diaphysären und metaphysären Abschnitt der Prothese liegt.

Während sich der diaphysäre Abschnitt 4 des Schafts an der starken Knochenrinde der Diaphyse abstützen kann und dort eine primäre Verankerung bewirkt, liegt der metaphysäre Abschnitt hauptsächlich im spongiösen Knochengewebe der Metaphyse.

In Fig. 2 erkennt man eine Stelle 7 mit der in AP-Richtung (anterior-posterior) größten Dimension des Schafts 3. Die Verbindung der Stellen größter AP-Dimension erscheint in Fig. 2 als Linie 7. Sie erstreckt sich über die gesamte Prothesenlänge. Für die Erfindung ist lediglich ihr Verlauf im metaphysären Abschnitt 5 von Interesse. Lateral von dieser Linie besteht die Oberfläche des Schafts im Ausführungsbeispiel der Fig. 3 ausschließlich aus Flächenanteilen, bei denen die Flächennormale eine in die Lateralrichtung weisende Komponente aufweist. Das bedeutet, daß mit herkömmlichen Mitteln keine Verbindung zwischen diesen Flächen und dem Knochen erreichbar ist, die zur Aufnahme von nach medial gerichteten Prothesenkräften fähig sind. Diese Kräfte setzen nämlich eine Zugverbindung zwischen diesen Oberflächenbereichen und dem Knochen voraus. In den Ausführungsbeispielen gemäß Fig. 4 oder 5 sind lateral von der Stelle bzw. Linie 7 auch hinterschnittene Flächenanteile vertreten, bei denen die Flächennormale keine in die Lateralrichtung weisende Komponente aufweist. Jedoch überwiegen auch hier die nach lateral blickenden Flächenanteile.

Ein im metaphysären Abschnitt (also oberhalb der Linie 8) und lateral von der Linie 7 liegender Teil 6 der Schaftoberfläche ist erfindungsgemäß mit einer osteoinduktiven Substanz ausgerüstet. Er ist in der Zeichnung durch eine Punktierung gekennzeichnet.Die Ausrüstung ist vorzugsweise in einer Schicht aus osteokonduktivem Material wie Hydroxylapatit enthalten. Durch diese Ausrüstung gelingt es, die Verbindung zwischen den betreffenen Oberflächenteilen und dem Knochengewebe so zu intensivieren, daß sie Zugkräfte zu übertragen vermag. Das bedeutet, daß die Zugtrajektorien im spongiösen Knochengewebe an der Kraftaufnahme beteiligt werden und demzufolge nicht abgebaut werden.

Der erfindungsgemäß ausgerüstete Bereich schließt im Ausführungsbeispiel der Fig. 1 einen Vorsprung 9 ein, der im trochantären Bereich der Metaphyse liegt. Derartige trochantäre Vorsprünge sind üblich, um die Verankerung des Schafts im Knochengewebe zu verbessern und den Schaft an einer Verdrehung im Verhältnis zum Knochen zu hindern.

Eine die Verbindung mit dem Knochen fördernde Oberflächengestaltung kann auch in den übrigen Bereichen des Schafts 3, d.h. medial von der Linie 7 und distal von der Linie 8, vorgesehen sein, beispielsweise eine Beschichtung mit Hydroxylapatit oder Kalziumphosphat. Jedoch soll sie dort keine osteoinduktiven Bestandteile enthalten, weil andernfalls die Entfernung des Prothesenschafts aus dem Knochen im Falle einer Reoperation zu sehr erschwert wird.

Fig. 2 veranschaulicht, daß der Vorsprung 13 eine beträchtliche Dicke in antero-posteriorer Richtung aufweist. Seine anterioren und posterioren Oberflächenanteile 6 sind daher dem mittleren Bereich entrückt, in welchem die spongiöse Knochensubstanz in manchen Fällen verarmt ist, und befinden sich in einem der Knochenrinde näheren und dichteren Bereich. Damit wird die Wahrscheinlichkeit einer guten Verbindung zwischen der Knochenoberfläche und der Knochensubstanz weiter vergrößert.

Fig. 5 veranschaulicht die Querschnittsform des Vorsprungs 9 in der Schnittrichtung V-V, die auch etwa der Einsetzrichtung entspricht. Wenn die Hohlform, die mittels einer Raspel zur Aufnahme der Prothese vorbereitet wurde, etwas knapper als die Prothesenform ist, ist mit dem Einfügen dieser Keilform in den Knochen eine Verdrängung von Knochensubstanz und dadurch eine Erhöhung der von der Knochensubstanz auf die Prothesenoberfläche ausgeübten Pressung verbunden. Auch dadurch wird ein rasch wachsender und inniger Verbund der Prothesenoberfläche mit dem Knochen gefördert.

Damit die innige Verbindung zwischen der Oberfläche des trochantären Vorsprungs und dem Knochen im Falle einer Revision nicht hinderlich ist, kann der Vorsprung 9 vom Schaft 3 lösbar sein. Beispielsweise kann er mittels Schrauben oder anderen Verbindungsmitteln mit dem Schaft 3 verbunden sein und von diesem gelöst werden, bevor der Schaft dem Knochen entnommen wird. Anschließend kann der Vorsprung leichter aus dem ihn umgebenden und mit ihm verwachsenen Knochen herausgelöst werden.

Die erfindungsgemäße Oberflächenausrüstung ist nicht an die in Fig. 1 und 2 dargestellte Prothesenform gebunden. Beispiele für andere Querschnittsformen zeigen Fig. 3 und 4. Die Bezugsziffer 7 weist dort auf den Querschnittspunkt größter Dicke in AP-Richtung, lateral von welchem die Erfindung eine osteoinduktive Ausrüstung der Prothesenoberfläche zuläßt.

## Patentansprüche

1. Hüftgelenkprothese mit einem in den Oberschenkelknochen einzusetzenden Schaft (3), dessen Oberfläche eine osteoinduktive Ausrüstung aufweist, **dadurch gekennzeichnet, daß** die osteoinduktive Ausrüstung ausschließlich in einem lateral von der Linie (7) größter AP-Abmessung des Schaftquerschnitts im metaphysären Abschnitt (5) liegenden Teil (6) des Schafts (3) vorgesehen ist.

2. Hüftgelenkprothese nach Anspruch 1, **dadurch gekennzeichnet, daß** die osteoinduktive Substanz von einer Beschichtung gebildet oder in einer Beschichtung enthalten ist.

3. Hüftgelenkprothese nach Anspruch 1 oder 2, **dadurch gekennzeichnet, daß** die osteoinduktive Substanz ein Bisphosphonat oder ein BMP umfaßt.

4. Hüftgelenkprothese nach Anspruch 1 oder 2, **dadurch gekennzeichnet, daß** mindestens der die osteoinduktive Substanz enthaltende Teil (6) der Prothesenoberfläche porös ist.

5. Hüftgelenkprothese nach Anspruch 1 oder 2, **dadurch gekennzeichnet, daß** der mit einer osteoinduktiven Substanz ausgerüstete Teil (6) der Prothese mindestens zwei einander ventral und eine dorsal gegenüberstehende Flächen umfaßt, die einen Abstand in AP-Richtung von mehr als 6 mm, vorzugsweise mehr als 9 mm einschließen.

6. Hüftgelenkprothese nach einem der Ansprüche 1 bis 6,**dadurch gekennzeichnet, daß** der mit einer osteoinduktiven Substanz ausgerüstete Teil (6) der Prothese mindestens teilweise von einem vom Schaft (12) abstehenden Vorsprung (13) gebildet ist.

7. Hüftgelenkprothese nach Anspruch 7, **dadurch gekennzeichnet, daß** der Vorsprung (13) in Implantationsrichtung keilförmig ist und eine der Prothese zugeordnete Raspel im Bereich dieses Vorsprungs ein geringeres Volumen aufweist.

8. Hüftgelenkprothese nach Anspruch 8, **dadurch gekennzeichnet, daß** der Vorsprung (13) vom Schaft lösbar ist.
